(19) Europäisches Patentamt
European Patent Office
Office européen des brevets

(11) Publication number: **0 405 998 A1**

# (12) EUROPEAN PATENT APPLICATION

(21) Application number: **90307125.6**

(51) Int. Cl.⁵: **C07K 7/56**, A61K 37/02

(22) Date of filing: **29.06.90**

The applicant has filed a statement in accordance with Rule 28 (4) EPC (issue of a sample only to an expert). Accession number(s) of the deposit(s):

(30) Priority: **30.06.89 US 374418**
**16.03.90 US 495019**

(43) Date of publication of application:
**02.01.91 Bulletin 91/01**

(84) Designated Contracting States:
**CH DE FR GB IT LI NL**

(71) Applicant: **MERCK & CO. INC.NC.**
**126, East Lincoln Avenue P.O. Box 2000**
**Rahway New Jersey 07065-0900(US)**

(72) Inventor: **Giacobbe, Robert A.**
**230 Westmont Avenue**
**Lavallette, NJ 08735(US)**
Inventor: **Hensens, Otto D.**
**64 Alexander Drive**
**River Plaza, Red Bank, NJ 07701(US)**
Inventor: **Liesch, Jerrold**
**10 Sherbrooke Drive**
**Princeton Junction, NJ 08550(US)**
Inventor: **Schwartz, Robert E.**
**765 Summit Avenue**
**Westfield, NJ 07090(US)**
Inventor: **Schmatz, Dennis M.**
**306 Elizabeth Avenue**
**Cranford, NJ 07016(US)**

(74) Representative: **Hesketh, Alan, Dr. et al**
**European Patent Department Merck & Co.,**
**Inc. Terlings Park Eastwick Roadoad**
**Harlow Essex, CM20 2QR(GB)**

## (54) Antibiotic agents.

(57) New antibiotic agents produced by the cultivation of Zalerion arboricola are described. The agents are a cyclic lipopeptides with very high activity against human pathogens and of very low mammalian toxicity.

FIG-1

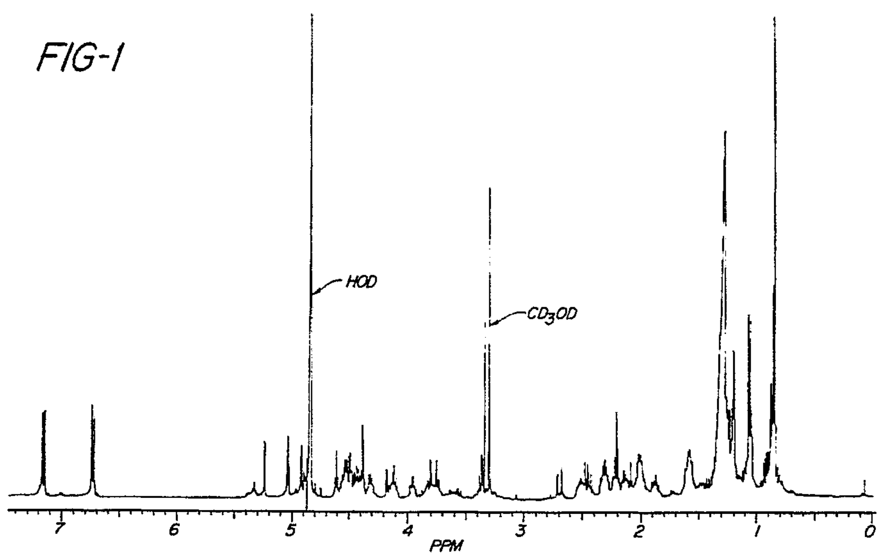


EP 0 405 998 A1

## ANTIBIOTIC AGENT

## ANTIBIOTIC AGENT

According to the present invention, three new closely related substances have been discovered on the cultivation of Zalerion arboricola which have been found to be useful as antifungal agents and as antipneumocystis agents.

The new compounds may be represented by the formula:

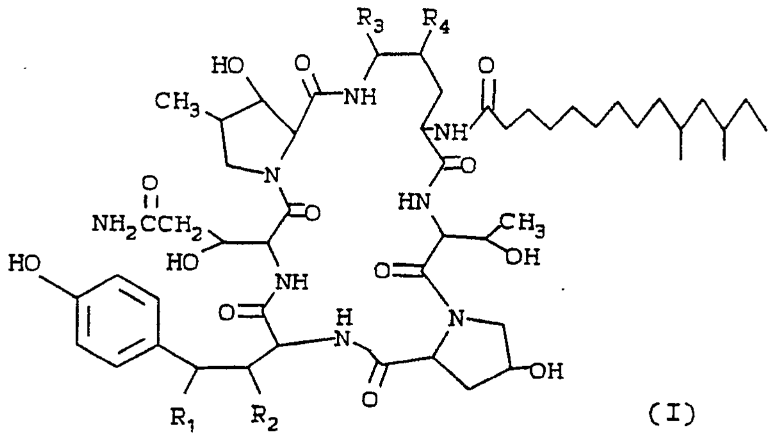

(I)

wherein $R_1$, $R_2$, $R_3$ and $R_4$ are hydrogen or hydroxy and are selected from one of the following compounds:

(1) $R_1$, $R_3$ and $R_4$ are hydroxy and $R_2$ is hydrogen, wherein the compound is designated Compound IA and may be named 1-[4,5-dihydroxy-$N^2$-(10,12-dimethyl-1-oxotetradecyl)-ornithine]-4-[4-hydroxy-4-(4-hydroxyphenyl)-2-aminobutanoic acid]-5-(3-hydroxyglutamine)echinocandin B.

(2) $R_1$, $R_2$ and $R_4$ are hydrogen, $R_3$ is hydroxy wherein the compound is designated Compound IB and may be named: 1-[5-hydroxy $N^2$-(10, 12-dimethyl-1-oxotetra-decyl)-ornithine]-4-[4-(4-hydroxyphenyl)-2-amino-butanoic acid]-5-(3-hydroxyglutamine) echinocandin B.

(3) $R_1$, $R_2$, $R_3$ and $R_4$ are hydrogen, wherein the compound is designated Compound IC and may be named 1-[$N^2$-(10,12-dimethyl-1-oxotetradecyl)ornithine]-4-[4-(4-hydroxyphenyl)-2-aminobutanoic acid]-5-(3-hydroxyglutamine)echinocandin B.

When the expression "Compound I" is hereinafter employed, without the letters A, B, or C, it is meant to embrace any compound represented by formula (I) or a mixture thereof. (In the formulas, a hydrogen attached to a carbon is not specifically designated unless it is one of $R_1$, $R_2$, $R_3$ or $R_4$.)

The present invention also embraces methods for inhibiting fungal growth and/or inhibiting or alleviating Pneumocystis carinii infections.

The structure of the compounds have been determined by detailed analyses of the spectral characteristics.

### MASS SPECTRAL DATA

Electron impact (EI) mass spectral data were obtained on a Finnigan-MAT 212 mass spectrometer at 90 eV. Gas Chromatograph-Mass Spectrogram (GC-MS) analyses of the TMS (trimethylsilyl) derivatives of total acid hydrolysates were performed on the same instrument. Fast atom bombardment (FAB) mass spectra were recorded on a VG20-253 or Finnegan MAT MAT90 instrument.

Compound IA has a molecular weight 1062 by FAB-MS (observed $(M+Na)^+$ at m/z 1085). GC-MS of the total acid hydrolysate disclosed one equivalent each of threonine, 4-hydroxyproline, 3-hydroxy-4-methylproline, 3-hydroxyglutamic acid, and C16:10 saturated fatty acid. The HR-FAB-MS from $[M+Cs]^+$ indicates the molecular formula to be $C_{51}H_{82}N_8O_{16}$ (Calcd 1062.5849, found 1062.5717).

Compound IB has a molecular weight 1030 by FAB-MS (observed $(M+Na)^+$ at m/z 1053). GC-MS of the total acid hydrolysate disclosed one equivalent each of threonine, 4-hydroxyproline, 3-hydroxy-4-methylproline, 3-hydroxyglutamic acid, and C16:O saturated fatty acid. The HR-FAB-MS from $[M+Cs]^+$ ion indicates the molecular formula to be $C_{51}H_{82}N_8O_{14}$ (Calcd 1030.5950, Found 1030.5829).

Compound IC has a molecular weight 1014 by FAB-MS (observed $(M+Na)^+$ at m/z 1037). GC-MS of

the total acid hydrolysate disclosed one equivalent each of threonine, 4-hydroxyproline, 3-hydroxy-4-methylproline, 3-hydroxyglutamic acid, and a C16:O saturated fatty acid. The HR-FAB-MS from $[M+Cs]^+$ ion indicates the molecular formula to be $C_{51}H_{82}N_8O_{13}$ (Calcd. 1014.6001, Found 1014.5915.)

NMR SPECTRAL DATA

$^1H$ and $^{13}C$ NMR spectra were recorded in $CD_3OD$ at ambient temperature on Vamian XL300 and XL400 spectrometers Chemical shifts are indicated in upper relative to TMS at zero ppm using the solvent peak at δ3.30 and 49.0 ppm as internal standards for $^1H$ and $^{13}C$ NMR spectra respectively. Compound IA $^1H$ NMR spectrum : in $CD_3OD$ at 400 MHz is as seen in Figure 1; $^{13}C$ NMR Chemical Shifts : obtained in $CD_3OD$ at 75 MHz 11.2, 11.6, 19.6, 20.2, 20.7, 27.0, 28.0, 30.30, 30.34, 30.6, 30.8, 31.2, 31.3, 32.9, 34.9, 36.7, 38.1, 38.7, 39.1, 39.9, 42.4, 45.9, 51.6, 52.7, 53.0, 55.5, 57.1, 58.4, 62.2, 68.7, 70.1, 70.6, 70.9, 71.3, 72.6, 74.3, 75.8, 116.2(2X), 128.5(2X), 136.1, 158.0, 169.4, 172.6(2X), 173.1, 173.8, 174.6, 175.9, 176.8 ppm.

Compound IB

$^1$ H NMR Spectrum in $CD_3OD$ at 400 MHz is seen in Figure 2.

Compound IC

$^1$ H NMR Spectrum in $CD_3OD$ at 400 MHz is seen in Figure 3.

$^{13}C$ NMR Chemical Shifts : obtained in $CD_3OD$ at 100 MHz.

11.2, 11.6, 19.8, 20.2, 20.7, 24.6, 27.0, 28.0(2x), 30.27, 30.33, 30.5, 30.7, 31.1, 31.2, 32.9(2x), 33.9, 36.7, 38.0, 38.3, 38.5, 39.0, 40.1, 45.9, 52.8, 53.0, 55.2, 55.7, 57.1, 58.6, 62.1, 68.2, 70.3, 70.7, 71.3, 76.0, 116.3-(2x), 130.5(2x), 133.0, 156.7, 170.2, 172.2, 172.7, 173.60, 173.63, 175.2, 176.3, 176.5 ppm

On the basis of these and other data, Compounds IA, IB, and IC are believed with considerable certainty to have the structures indicated.

The compounds are white solids, soluble in organic solvents such as methanol, ethanol, dimethylformamide, dimethyl sulfoxide, ethyl acetate and the like.

The compounds of this invention have antifungal properties against both filamentous fungi and yeasts. They are particularly useful against organisms causing pathogenic mycotic infections such as Candida albicans , Candida rugosa , Candida parapsilosis and the like, where high activity is exhibited over a panel of strains of the organisms.

Moreover, unlike a number of antifungal agents, such as amphotericin B, which while active against Candida albicans and other fungal pathogens are limited in their ability because of the untoward and dangerous side effects, the antifungal agents of the present invention are not only very effective but are substantially free of undesirable side reactions.

Red blood cell lysis, a harmful and potentially fatal side reaction is shown by many compounds at concentrations approaching the therapeutic dose and this property has limited the applicability of these compounds as drugs. The compounds of the present invention would require a concentration of drug far above that required for therapeutic use before red blood cell lysis could occur.

The compounds are also effective antifungally against filamentous fungi, especially Cochliobolus miyabeanus , Aspergillus species, Penicillium species, Fusarium species, Alternaria species, Neurospora species and the like.

The compounds of formula (I) are also useful as an agent for the treatment of Pneumocystis carinii , the causative agent of pneumonia of particular severity to immune compromised patients such as those with acquired immune deficiency syndrome (AIDS).

The compounds of the present invention, represent by formula (I) are conveniently produced by cultivating MF 5171 Zalerion arboricola , ATCC No. 20868, deposited under the Budapest Treaty in the Culture Collection of the American Type Culture Collection at 12301 Parklawn Drive, Rockville, MD 20852.

The colonial and morphological description of MF 5171, ATCC 20868 are set forth below:

Colonies on potato-dextrose agar (Difco) at 20 C slow-growing, attaining a diameter of 8-12 mm in one week. Mature colonies (3-4 weeks) on potato-dextrose agar effuse, with submerged and aerial hyphae, surface hairy, lanose, or funiculose, dull to moderately shiny, forming raised, densely compact colonies, with a substromatic texture due to dense conidia formation. Colony color pale olive-brown, olive, olive-brown finally olive-black, Isabella Color, Sayal Brown, Tawny-olive, Saccardo's Umber, Sepia, Brownish Olive, Raw Umber, Dark Olive, Olivaceous Black (capitalized color names from R. Ridgway. 1912. Color Standards and Nomenclature, Washington, D.C.). Same colors in colony reverse. Odor, exudates, and soluble pigments absent.

Hyphae (in 3% KOH) pale yellow-brown to olive-brown, septate, branched, often with irregular lateral or terminal lobes, 1-3 um wide, thin- to slightly thick-walled, with walls smooth to slightly incrusted or verrucose. Aerial hyphae often adhering together in fascicles. Setae and hyphopodia absent.

Conidiogenous cells monoblastic, scattered to denfe, integrated, terminal and intercalary, arising dierctly from undifferentiated hyphae, at right to slightly acute angles. Conidia originating as irregular chains,

filaments, or coils, later developing as compact, irregular masses of 6-25 cells. Individual conidial cells, 3-6 um in diameter, globose, subglobose, or slightly irregular to lobed, smooth to finely verruculose, yellow-brown to olive brown.

Although the production of the novel compound is discussed hereinbelow principally with respect to a specific strain, ATCC 20868, all strains of the genus Zalerion arboricola and mutants are contemplated within the scope of this invention.

However, one particular mutant identified in the Merck Culture Collection as MF 5416 and accessible from the American Type Culture Collection as Z. arboricola ATCC 20988 is useful in the production of Compound IC.

Compound I may be produced by cultivating Zalerion arboricola in a suitable nutrient medium under conditions hereinafter described until a substantial amount of antifungal activity is detected in the culture medium, harvesting by extracting the active components from the fermentation medium with a suitable solvent, concentrating the solution containing the desired component, then subjecting the concentrated material to chromatographic separation to isolate Compound I from other metabolites also present in the cultivation medium.

The compound of formula (X), (hereinafter Compound X).

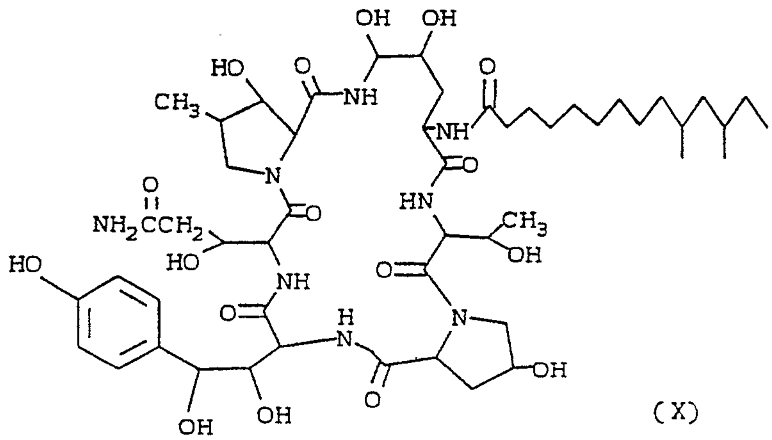

(X)

which is the subject of copending application S.N. 362,647, filed June 7, 1989, which is a continuation application of Serial No. 105,795, corresponding to EP 311193 filed October 10, 1987, now abandoned, and named therein as 1[4,5-dihydroxy-$N^2$-(10,12-dimethyl-1-oxotetradecyl)ornithine]-5-(3-hydroxyglutamine)-echinocandin B also is produced by the organism in significant amounts. Therefore in order to favor the production of Compound I, a modified nutrient medium hereinafter described is employed.

## PRODUCTION

The production of the compounds of the present invention may be carried out by cultivating Zalerion arboricola in a suitable nutrient medium. The fermentation is carried out in a medium containing sources of carbon and nitrogen assimilable by the microorganism and also containing low levels of inorganic salts. In addition, the medium may be supplemented with trace metals, although if complex sources of carbon and nitrogen are employed, the trace metals are usually present in the complex sources.

The sources of carbon include glycerol, sugars, sugar alcohols, starches and other carbohydrates, or carbohydrate derivatives such as dextran, cerelose, as well as complex nutrients such as oat flour, corn meal, millet, corn and the like. The exact quantity of the carbon source which is utilized in the medium will depend, in part, upon the other ingredients in the medium, but it is usually found that an amount of carbohydrate between 0.5 and 40 percent by weight of the medium is satisfactory. These carbon sources can be used individually or several such carbon sources may be combined in the same medium.

The sources of nitrogen include amino acids such as glycine, arginine, threonine, methionine and the like, ammonium salt, as well as complex sources such as yeast hydrolysates, yeast autolysates, yeast cells, tomato paste, soybean meal, casein hydrolysates, yeast extracts, corn steep liquors, distillers solubles, cottonseed meal, meat extract, and the like. The various sources of nitrogen can be used alone or in combination in amounts ranging from 0.2 to 10 per cent by weight of the medium.

Among the nutrient inorganic salts, which can be incorporated in the culture media are the customary salts capable of yielding sodium, potassium, magnesium, calcium, phosphate, sulfate, chloride, carbonate, and like ions. Also included are trace metals such as cobalt, manganese, iron, molybdenum, zinc, cadmium, and the like.

Although the growth medium may be prepared from conventional materials such as the foregoing, the presence of certain nutrients are important, and particularly certain combinations subsequently detailed favor the production of Compound I relative to Compound X. The presence of ammonium salts and monobasic potassium phosphate is important, the former as immediate source of nitrogen and the latter for pH control. While the carbon sources may be as above listed, the amount of the desired product is enhanced by the presence of glycerol or mannitol. Illustrative of a glycerol containing medium is the following:

| Medium I | |
|---|---|
| Glycerol | 85.0 g |
| Pectin | 10.0 g |
| Peanut Meal | 4.0 g |
| Peptonized Milk | 4.0 g |
| Tomato Paste | 4.0 g |
| Corn Steep Liquor | 4.0 g |
| Lard Water | 4.0 g |
| Glycine | 2.0 g |
| $KH_2PO_4$ | 2.0 g |
| Distilled Water | 1000.0 ml |
| Pre Sterile pH = 7.0 | |

Moreover, the rate of production may be facilitated several-fold by the substitution of mannitol for glycerol.

Representatives suitable mannitol containing media for production of Compound I are the following:

| RG2 MEDIUM | | RG120 MEDIUM | |
|---|---|---|---|
| | per liter | | per liter |
| Mannitol | 44 g | Mannitol | 91 g |
| Corn Steep Liquor | 4 g | Corn Steep Liquor | 4 ml |
| Lard Water | 4 g | Lard Water | 4 g |
| Pectin | 10 g | Pectin | 10 g |
| $KH_2PO_4$ | 2 g | $KH_2PO_4$ | 2 g |
| Tomato Paste | 4 g | Tomato Paste | 4 g |
| Peptonized Milk | 4 g | Peptonized Milk | 4 g |
| Glycine | 2 g | Glycine | 2 g |
| Peanut Meal | 4 g | Peanut Meal | 4 g |
| pH adjusted to | 7.0 | pH adjusted to | 7.0 |

Moreover, the rate of production may be increased by use of mannitol in an amount of at least 4% by weight of medium with careful pH control at about 5.5. Representative of such media are those designated TG102 and TG103.

| TG102 MEDIUM | per liter | TG103 MEDIUM | per liter |
|---|---|---|---|
| D-Mannitol | 40 g | D-Mannitol | 40 g |
| Bacto-Peptone* | 33 g | Nz Amine (Type E) | 33 g |
| Bacto-Yeast Extract | 10 g | Fidco Yeast Extract | 10 g |
| $(NH_4)_2SO_4$ | 5 g | $(NH_4)_2SO_4$ | 5 g |
| $KH_2PO_4$ | 9 g | $KH_2PO_4$ | 9 g |
| no pH adjustment | | no pH adjustment | |

* Casein hydroysate - Humko-Sheffield, Memphis, Tenn.

While Compound I may be produced employing the foregoing liquid media, the amounts are relatively small and with some media only some of the compounds are formed. It has been found that by formulating media employing peptonized milk as the sole complex nitrogen source, liquid growth media may be devised which significantly increases the yields of all the compounds of the present invention. Thus, a medium which contains D-mannitol as carbon source, peptonized milk as the complex nitrogen source, glycine as amino acid and a buffer, and preferably containing lactic acid, trace elements and vegetable oil selected from soy, peanut and sunflower oils, has been the most satisfactory for obtaining best yields of the compounds of the present invention.

The useful ranges for the components are as follows:

| | grams/liter |
|---|---|
| D-mannitol | 20-100 |
| $KH_2PO_4$ | 0.5-3 |
| glycine | 1-4 |
| Peptonized milk | 2-20 |
| Lactic acid | 0-3 |
| Trace element mixture | 0-15 ml |
| Vegetable oil (soy, peanut, sunflower) | 0-20 |
| presterilization pH = 7 | |
| Trace elements | per liter 0.6NHCl |
| $FeSO_4 \cdot 7H_2O$ | 1.0 g |
| $MnSO_4 \cdot 4H_2O$ | 1.0 g |
| $CuCl_2 \cdot 2H_2O$ | 0.025 g |
| $CaCl_2$ | 0.1 g |
| $H_3BO_3$ | 0.056 g |
| $(NH_4)_6Mo_7O_{24} \cdot H_2O$ | 0.019 g |
| $ZnSO_4.7H_2O$ | 0.2 g |

For best yields of the compound, all of the components of the foregoing composition should be present. Thus, S6 medium below is a preferred medium.

| S2 MEDIUM | | S6 MEDIUM | |
|---|---|---|---|
| | per liter | | per liter |
| D-Mannitol | 44 g | D-Mannitol | 44 g |
| $KH_2PO_4$ | 2 g | $KH_2PO_4$ | 2 g |
| Glycine | 2 g | Glycine | 2 g |
| Peptonized Milk | 15 g | Peptonized Milk | 15 g |
| Lactic acid | 2 g | Lactic acid | 10 ml |
| Trace Elements | 10 ml | Trace Elements | 10 g |
| | | Soybean oil | 10 g |
| pH 7.0 (pre-sterilization) | | pH 7.0 (pre-sterilization) | |

In addition to the liquid media, the following solid media may be employed:

| F204 SOLID MEDIUM | | | |
|---|---|---|---|
| | per 250-ml flask | Base liquid | per liter |
| Millet | 15 g | Ardamine PH (**) | 33.0 g |
| Base liquid | 15 ml | Sodium Tartrate | 6.6 g |
| | | $FeSO_4 \cdot 7H_2O$ | 0.66 g |
| | | Monosodium Glutamate | 6.6 ml |
| | | Corn oil | 6.6 ml |
| | | no pH adjustment | |

**Yeast autolysate available from Yeast Products Inc. Clifton, New Jersey

| F4-SF SOLID MEDIUM | | | |
|---|---|---|---|
| | per 250-ml flask | Base liquid | per liter |
| Cracked corn | 15 g | Ardamine PH | 0.2 g |
| Base liquid | 10 ml | $KH_2PO_4$ | 0.1 g |
| | | $MgSO_4 \cdot 7H_2O$ | 0.1 g |
| | | Sodium Tartrate | 0.1 g |
| | | $FeSO_4 \cdot 7H_2O$ | 0.01g |
| | | $ZnSO_4 \cdot 7H_2O$ | 0.01g |
| | | no pH adjustment | |

The foregoing media may be employed in fermentations carried out using a standard procedure wherein first a frozen vegetative mycelia of Zalerion arboricola (ATCC 20868) are thawed and used to inoculate a seed medium which is then incubated for a minimum of 3 days to produce organisms which serve as seeds in the production of the compounds of formula (I). The seed medium may have the following composition:

| Seed Medium (KF Medium) | |
|---|---|
| | per liter |
| Corn Steep liquor | 5.0 g |
| Tomato paste | 40.0 g |
| Oat flour | 10.0 g |
| Glucose | 10.0 g |
| Trace elements | 10.0 ml |
| Distilled water | to 1000 ml |
| pH 6.8 | |

In this process, a slant section of a preserved oulture of ATCC 20868 is inoculated into an appropriate liquid nutrient seed medium of pH in the range 5 to 8.1, optimally 6 to 7.5, and the flasks incubated with or without agitation at temperatures in the range of from about 15 °C to about 30 °C, preferably 20 ° to 28 °C. Agitation when employed, may be up to 400 rpm, preferably, about 200 to 220 rpm. The incubation is carried out over a period of from 2 to 30 days, preferably 2 to 14 days. When growth is abundant, usually between 2 and 5 days, the growth may be used to inoculate the production medium for the production of the compounds of this invention. Preferably however a second stage fermentation is carried out, inoculating with a portion of the culture growth and then employing similar conditions but generally with a shortened incubation period of about 1 to 3 days. The growth then is employed to inoculate the production medium.

The fermentation production medium inoculated with the culture growth is incubated for 3 to 30 days, usually 7 to 14 days, with or without agitation. The fermentation may be conducted aerobically at temperatures ranging from about 20 °C to about 40 °C. For optimum results, it is most convenient to conduct these fermentations at a temperature in the range of from about 24 °C to about 30 °C. Temperatures of about 24 °-28 °C are most preferred. The pH of the nutrient medium suitable for producing the instant compounds can vary from about 5.0 to 8.5 with a preferred range of from about 6.0 to 7.5. After the appropriate period for the production of the desired compound or compounds, the latter is recovered from the fermentation medium as hereinafter more fully described.

## HARVEST AND ISOLATION

After production by cultivation employing one of the above-described methods, Compound I is harvested and isolated from the fermentation medium.

The exact steps may vary somewhat depending on whether the fermentation is carried out in liquid or solid medium, what solvent is employed and what adsorbent or combination of adsorbents is employed.

When the fermentation is carried out on a solid medium, the first step may be carried out by adding an alcoholic solvent to the fermentation medium, thoroughly mixing, then filtering, recovering and concentrating the aqueous alcohol filtrate. The concentrated filtrate may be first back-extracted or washed with a lower aliphatic hydrocarbon solvent such as hexane or other alkane to remove alkane soluble impurities. The alkane washed filtrate may be extracted or partitioned with a water-immiscible oxygenated organic solvent and the resulting solution concentrated, then placed onto a column for at least one, generally several chromatographic separation steps. Suitable columns are silica gel, reverse phase resin and "Sephadex" LH-20 (dextran adsorbent, Pharmacia).

When the fermentation is carried out in a liquid medium, in one method, the mycelial solids are filtered and recovered from the fermentation medium. Alcohol is added to the mycelial cake, and the mycelial solid thoroughly mixed with the alcohol, filtered, and the filtrate collected and concentrated. In an alternative method, the whole broth can be extracted by the addition of one volume of alkanol, preferably methanol, and filtered to remove solid impurities. The alkanol extract is then adsorbed on "Diaion" HP-20 (Mitsubishi Chemical Industries, Ltd) resin and eluted with 100% alkanol. "Diaion" SP-207 (brominated) or other commercially available styrene-divinylbenzene copolymer also may be employed. A second dilution/HP-20 adsorption/elution step is utilized to concentrate the sample in preparation for chromatographic separations. Sometimes, a third dilution/HP-20 adsorption/elution step may be desirable for volume reduction.

The alcoholic solvent to be employed in the initial extraction of the active agent from the solid nutrient medium or from the mycelial pad may be any of the lower alcohols such as methanol, ethanol, isopropanol,

and the like. Methanol is preferred.

If the active agent is partitioned from the alkanol or methanol solution, then suitable solvents are esters, such as ethyl acetate, isopropyl acetate, butyl acetate, or ketones, such as methyl ethyl ketone.

The chromatographic separation may be carried out by employing conventional column chromatography with non-ionic resin or by high performance liquid chromatography employing reverse phase resin. The fractions containing the antibiotic Compound I may be detected by antifungal assay using Candida albicans . Generally, more than one chromatographic separation steps are employed. In a most preferred procedure, one or more separations are carried out employing column chromatography and a final separation is carried out employing high performance liquid chromatography (HPLC) with $C_{18}$ reverse phase resin.

When conventional column chromatography is employed for chromatographic separations, silica gel is the preferred adsorbent. Usually more than one chromatographic separation is employed. Silica gel may be used in all the separations while employing different eluting agents. However, it may be combined advantageously with the use of a different adsorbent such as a dextran adsorbent sold under the trade name of "Sephadex" LH-20. Other adsorbents such as alumina, styrene-divinylbenzene copolymers available commercially as "Diaion" HP-20, HP-30, HP-40, SP-207 and "Amberlite" XAD-2, XAD-4, XAD-16 (Rohm and Haas Co.) also may be employed.

In the fractionation and recovery of the active component by chromatography on silica gel, ester/alcohol mixtures with increasing concentration of alcohol provide good separations. A mixture of ethyl acetate and methanol has been found to be especially useful. These may be employed in isocratic, step gradient or continuous gradient systems. When a dextran adsorbent such as "Sephadex" LH-20, is employed, a chlorohydrocarbon/hydrocarbon/alcohol solvent system may be employed. A mixture of methylene chloride/hexane/methanol has been found to be especially useful.

In carrying out the HPLC separation, the alcohol solution containing material recovered from the conventional chromatography is concentrated and the residue dissolved in methanol/water in the same ratio as found in the mobile phase and placed on a column packed with commercial reverse phase resin or on a column filled with silica gel/$C_{18}$ reverse phase resin prepared as amply reported in the literature. Alternatively, the alcohol solution may be diluted to 50 percent with water and pumped onto the column. The column is operated using methanol/water (1:1 or optionally other ratios) at 800-2000 psi which produces a flow rate of about 20 ml/min. Separation is monitored at 210 nm.

The fractions are assayed for activity with Candida albicans . The product is recovered from any of the chromatographic procedures by combining the Candida albicans active fractions and concentrating under reduced pressure.

The superior properties of Compound I as a therapeutic agent in the treatment of mycotic infections may be illustrated with minimum fungicidal concentration (MFC) results in tests against Candida albicans , Candida tropicalis and Candida parapsilosis .

The activity may be seen in a microbroth dilution assay employing as medium a Yeast Nitrogen Base (Difco) with 1% dextrose (YNBD) as the medium. In carrying out the assay, Compound IB, IC-1 and IC-2 were solubilized in 10 percent dimethyl sulfoxide (DMSO) and diluted to 2560 μg/ml. The compounds were then diluted to 256 μg/ml in YNBD. 0.15 ml of the suspension was dispensed to the top row of a 96-well plate (each well containing 0.15 ml of YNDB) resulting in a drug concentration of 128 μg/ml. Two-fold dilutions were then made from the top row to obtain final drug concentrations ranging from 128 to 0.06 μg/ml.

The yeast cultures, maintained on Sabouraud dextrose agar were transferred to YM broth (Difco) and incubated overnight at 35 °C with shaking (250 rpm). After incubation, each culture was diluted in sterile water to yield a final concentration of 1-5 x $10^5$ colony forming units (CFU)/ml.

96-well microplates were inoculated using a MIC-2000 (Dynatech) which delivers 1.5 μl per well yielding a final inoculum per well of 1.5-7.5 x $10^3$ cells. The microplates were incubated at 35 °C for 24 hours. The minimum inhibitory concentrations (MICs) were recorded as the lowest concentrations of drug showing no visible growth.

After recording the MIC, the plates were shaken to resuspend the cells. Thereafter, 1.5 ml samples from the wells in the 96-well microplate were transferred to a single well tray containing Sabouraud dextrose agar. The inoculated trays were incubated 24 hours at 28 °C and then read. The MFC is defined as the lowest concentration of drug showing no growth or less than 4 colonies per spot.

| Fungi Strain No. | Minimum Fungicidal Concentration (µg/ml) | | |
|---|---|---|---|
| | IB | IC-1 | IC-2 |
| Candida albicans | | | |
| MY 1055 | 0.5 | NT | 0.5 |
| MY 1585 | 1.0 | 1.0 | 1.0 |
| MY 1208 | 2.0 | 4.0 | 2.0 |
| MY 1028 | 1.0 | 2.0 | 2.0 |
| MY 1750 | 1.0 | 0.5 | 1.0 |
| MY 1783 | 2.0 | 4.0 | 1.0 |
| Candida tropicalis | | | |
| MY 1012 | 2.0 | 4.0 | 4.0 |
| Candida parapsilosis | | | |
| MY 1009 | 16.0 | 128.0 | 16.0 |
| MY 1010 | 8.0 | 16.0 | 8.0 |

The foregoing results are merely exemplary of the superior and consistent antimycotic properties shown by Compound I.

The compound is also a broad spectrum antifungal agent effective against many fungal species, both filamentous fungi and yeasts.

Compound I or a component thereof has potential as a replacement for a known antifungal agent which while effective as an antifungal agent is of limited utility for having lytic effect on red blood cells at low dose levels. This property was discovered in a standard titration/hemolysis assay using fresh blood drawn from CD-1 female mice. In a representative assay, a concentration of Compound IB in the range of from 0.39 to 400 µg/ml in 5 percent dextrose and a drug free control were employed.

In carrying out the determination, the assay tubes prepared by mixing together 2 milliliters of appropriate drug dilution and 0.5 milliliter of red blood cell suspension were gently shaken to mix the contents and then the tubes were incubated at 25° C for two hours. At the end of this time, the tubes were examined visually for complete or partial red blood cell hemolysis and compared to a drug free control. The minimum lytic concentration (MLC) was taken as the highest drug concentration that did not lyse the red blood cells. The MLC for Compound IB was greater than 400 µg/ml. The same experiment carried out with amphotericin B showed the MLC for amphotericin B to be 12.5 µg/ml.

Compound I or one of the components is useful for inhibiting or alleviating Pneumocystis carinii infections. In such use, Compound I or component is administered in a therapeutically effective or inhibitory amount to subjects infected with or to immune compromised subjects susceptible to being infected with Pneumocystis carinii .

The efficacy of the compounds of the present invention for therapeutic or anti-infective purposes may be demonstrated in studies on immunosuppressed mice or rats.

In a representative study on the effectiveness of Compound IB, ten male C3H/He mice, weighing 22-24 gms. each, were immunosuppressed by the addition of dexamethasone to the drinking water (8.0 mg/L) for six weeks to induce the development of P. carinii infections. To enhance the infection the mice were also maintained on a low protein diet. At the beginning of the seventh week the mice were divided into two groups. Both groups continued to receive dexamethasone in the drinking water and low protein diet for the remainder of the study. Mice in Group I were injected intraperitoneally twice daily with 0.5 ml of a 10% dimethylsulfoxide (DMSO) solution as a vehicle control. Mice in Group II were injected intraperitoneally twice daily with 0.5 ml of sterile water containing 0.05 mg of compound IB (dissolved in DMSO, final concentration of DMSO is 10%, actual dose was 2.0 mg/kg). The treatment period lasted two weeks.

At the end of the treatment period (a total of eight weeks immunosuppression) the animals were sacrificed and the lung tissue removed. The tissue was then processed to determine the number of cysts for each animal. Compound IB reduced the number of cysts by 84% as compared to the control animals.

In a representative study on the effectiveness of Compound IC, twelve male C3H/He mice, weighing 22-24 gms. each, were immunosuppressed by the addition of dexamethasone to the drinking water (8.0 mg/L) for six weeks to induce the development of P . carinii infections. To enhance the infection the mice were also maintained on a low protein diet. At the beginning of the seventh week the mice were divided into two groups. Both groups continued to receive dexamethasone in the drinking water and low protein diet for the remainder of the study. Mice in Group I were injected intraperitoneally twice daily with 0.5 ml of a 10% DMSO solution as a vehicle control. Mice in Group II were injected intraperitoneally twice daily with 0.5 ml of sterile water containing 0.05 mg of Compound IC (dissolved in DMSO, final concentration of DMSO is 10%, actual dose was 2.0 mg /kg). The treatment period lasted one week.

At the end of the treatment period (a total of seven weeks immunosuppression) the animals were sacrificed and the lung tissue removed. The tissue was then processed to determine the number of cysts for each animal. Compound IC reduced the number of cysts by 66% as compared to the control animals.

The outstanding properties are most effectively utilized when the compound is formulated into novel pharmaceutical compositions with a pharmaceutically acceptable carrier according to conventional pharmaceutical compounding techniques.

The novel compositions contain at least a therapeutic antifungal or antipneumocystis amount of the active compound. Generally, the composition contain at least 1 percent by weight of Compound I or one of the components. Concentrate compositions suitable for dilutions prior to use may contain 90 percent or more by weight. The compositions include compositions suitable for oral, rectal, topical, parenteral (including subcutaneous, intramuscular, and intravenous), pulmonary (nasal or buccal inhalation), nasal administration, or insufflation. The compositions may be prepacked by intimately mixing Compound I with the components suitable for the medium desired.

When the compound is for antifungal use any method of administration may be used. For treating mycotic infection oral administration is frequently preferred. When oral administration is to be employed, it may be with a liquid composition or a solid composition. For liquid preparations, the therapeutic agent is formulated with liquid carriers such as water, glycols, oils, alcohols, and the like, and for solid preparations such as capsules and tablets, solid carriers such as starches, sugars, kaolin, ethyl cellulose, calcium and sodium carbonate, calcium phosphate, kaolin, talc, lactose, generally with lubricant such as calcium stearate, together with binders, disintegrating agents and the like. Because of their ease in administration, tablets and capsules represent the most advantageous oral dosage form. It is especially advantageous to formulate the compositions in unit dosage form (as hereinafter defined) for ease of administration and uniformity of dosage. Composition in unit dosage form constitutes an aspect of the present invention.

The Compound I also may be formulated in therapeutic compositions for intravenous or intraperitonal injection and may be presented in unit dosage form in ampoules or in multidose containers, if necessary with an added preservative. The compositions may also take such forms as suspensions, solutions or emulsions in oily or aqueous vehicles such as 0.85 percent sodium chloride or 5 percent dextrose in water, and may contain formulating agents such as suspending stabilizing and/or dispersing agents. Buffering agents as well as additives such as saline or glucose may be added to make the solutions isotonic. The drug also may be solubilized in alcohol/propylene glycol or polyethyleglycol for drip intravenous administration. Alternatively, the active ingredients may be in powder form for reconstituting with a suitable vehicle prior to administration.

The term "unit dosage form" as used in the specification and claims refer to physically discrete units, each unit containing a predetermined quantity of active ingredient calculated to produce the desired therapeutic effect in association with the pharmaceutical carrier. Examples of such unit dosage forms are tablets, capsules, pills, powder packets, wafers, measured units in ampoules or in multidose containers and the like. A unit dosage of the present invention will generally contain from 100 to 200 milligrams of one of the compounds.

When the compound is to be employed for control of pneumocystis infections it is desirable to directly treat lung and bronchi. For this reason, inhalation methods are preferred. For administration by inhalation, the compounds of the present invention are conveniently delivered in the form of an aerosol spray presentation from pressurized packs of nebulisers. The compounds may also be delivered as powders which may be formulated and the powder composition may be inhaled with the aid of an insufflation powder inhaler device. The preferred delivery system for inhalation is a metered dose inhalation (MDI) aerosol, which may be formulated as a suspension or solution of Compound I in suitable propellants, such as fluorocarbons or hydrocarbons.

Another method of administration is insufflation, particularly if the infection has spread to the ears and other body cavities.

If the application is to be topical, the drug may be formulated in conventional creams and ointments

such as white petrolatum, anhydrous lanolin, cetyl alcohol, cold cream, glyceryl monostearate, rose water and the like. Usually a 1 to 2 percent cream solution is prepared and applied to the area to be treated.

Compound I also exhibits broad spectrum antifungal activity against yeasts and filamentous fungi (molds). For non-medical application, the product of the present invention, may be employed in compositions in an inert-carrier which includes finely divided dry or liquid diluents, extenders, fillers, conditioners and excipients, including various clays, diatomaceous earth, talc, and the like, or water and various organic liquids such as lower alkanols, for example, ethanol and isopropanol, or kerosene, benzene, toluene and other petroleum distillate fractions or mixtures thereof. The antifungal compositions may be applied directly to areas where fungal control is desired.

The following examples illustrate the invention but are not to be construed as limiting:

## Example I

### Fermentation

The contents of one frozen vial of Zalerion arboricola ATCC 20868 from the Merck Culture Collection were defrosted and aseptically transferred to a 250 milliliter unbaffled flask containing 54 milliliters of medium containing 0.4 per cent agar. The modified medium after inoculation, was incubated at 25 °C with 220 rpm agitation for 48 hours. At the end of this period, 10 milliliters of the growth medium was transferred to a 2-liter unbaffled flask containing 500 milliliter of KF medium containing 0.4 per agar. After inoculation, the resulting medium was incubated for 24 hours at 25 °C with 220 rpm agitation.

Twenty 2-liter flasks each containing 120 grams of F204 (solid medium) and 120 milliliters of a stock solution consisting of:

| | |
|---|---|
| Yeast extract | 5 parts by weight |
| Sodium tartrate | 1 part by weight |
| Ferrous sulfate crystals | 0.1 part by weight |
| Monosodium glutamic acid | 1 part by weight |
| Corn Oil | 1 part by weight |

were autoclaved for 20 minutes at 122 °C and then reautoclaved with 80 milliliters of water for another 20 minutes at 122 °C. The flasks were allowed to cool, then inoculated with 20 milliliters of seed medium prepared as above described, and the inoculated flasks incubated at 25 °C under static conditions for 14 days.

### ISOLATION

One liter of methanol was added to each of nineteen 2-liter flasks of solid fermentation and the contents of the flasks were combined, stirred and filtered. The spent cake was then extracted twice with 6 liters of methanol, filtered, and the three filtrates combined and concentrated to 3 liters.

The three liter concentrate was diluted with one liter of water and extracted twice with 3 liters of ethyl acetate. The two 3-liter ethyl acetate extractions were combined, dried with sodium sulfate and concentrated to 100 milliliters.

The 100 milliliter concentrate was adsorbed onto silica gel by adding 100 milliliters of methanol and 100 milliliters of dry silica gel (EM Science, Silica Gel, 60-200 mesh) and removing the solvent on a rotary evaporator. The dried silica gel was then applied to a 500 ml silica (EM Science, Silica Gel, 60-200 mesh) gel column and eluted with ethyl acetate, followed by ethyl acetate/methanol (9:1). The eluates were tested for presence of antibiotic by determining activity against Candida albicans .

The antibiotic rich eluates were combined, concentrated to an oil and dissolved in 200 milliliters of methylene chloride/hexane/methanol (10:10:1) and combined with 40 ml "Sephadex" LH-20 which had been

prepared by soaking overnight in methanol and washing twice with 200 milliliters of 10:10:1 methylene chloride/hexane/methanol. After soaking for a few minutes, the supernatant was removed via filtration and the "Sephadex" LH-20 was washed with 200 milliliters of 10:10:1 methylene chloride/hexane/methanol and filtered. The filtrates were found not to contain active constituents and were discarded.

The active constituents which had partitioned into the "Sephadex" LH-20 beads were recovered by washing the beads twice with 200 milliliters of methanol, and thereafter combining and concentrating the methanol washes.

The combined concentrate was placed on 200 milliliters of silica gel (EM Science, Kieselgel 60, 230-400 mesh) and eluted with ethyl acetate/methanol (75:25) to recover in the eluate biologically active materials. 435 mg of the biologically active material was Compound X; 123 mg of the biologically active material was a mixture. The latter was dried onto 2 ml of silica gel and chromatographed on a 70 ml silica gel column using ethyl acetate/methanol in a step gradient elution. The biologically active component eluted with 50:50 ethyl acetate/methanol and amounted to 54 grams of a mixture.

This mixture was chromatographed on an 88 ml "Zorbax" ODS column (DuPont) using a solvent mixture of 50:50 acetonitrile/water at a flow rate of 20 ml/minute and monitoring at 210 nm. Fractions were collected and evaluated. Based on antifungal activity and analytical HPLC, fractions 11-14, having retention time in the range 9.23-9.27 minutes were combined and concentrated to obtain about 5 mg of the first compound; fractions 18-22, having a retention time of about 13.48-13.49 minutes when concentrated and combined amounted to about 5 mg and a second compound; and fractions 26-32 having a retention time of 18.39-18.40 when concentrated and combined amounted to about 5 mg of a third compound. NMR and mass spectral determinations were made on each of the compounds and on the basis of these determinations were assigned structures for Compound IA, IB and IC, respectively.

## Example II

Fifty milliters of KF seed medium in 250 milliters unbaffled Erlenmeyer flask was inoculated with a frozen vial.of Z arboricola ATCC 20868 and shaken at 220 rpm and 25° C for 72 hours. Two milliliters of this seed was used to inoculate 50 µl of KF medium and was grown as above for 72 hrs. The second stage was used to inoculate the production medium.

Forty milliliters of medium previously detailed as Medium S-6 in 250 µl unbaffled Erlenmeyer flasks was inoculated with 2 milliliters of seed and the flasks shaken at 220 rpm and 25° C for 14 days.

To the resulting broth was added an equal volume of methanol and the diluted broth placed in tubes. The tubes were shaken on a reciprocating shaker for 30 minutes and centrifuged at 3000 rpm for 15 minutes. The cell pellet was discarded and the methanol from the supernatant was removed in vacuo . To the aqueous phase was added an equal volume of methyl ethyl ketone (MEK) and the tubes shaken for 30 minutes on a reciprocating shaker. The phases were separated by centrifugation at 3000 rpm. The MEK layer was separated and the extraction of the aqueous phase repeated.

The MEK layers from the two extractions were combined and evaporated to dryness in vacuo . The residue was dissolved in a volume of methanol which was 1/10 of the original volume of the sample. This extraction analysed by HPLC. It was found that Compound I was produced.

## Example III

The contents of a vial of frozen vegetative mycelia of Zalerion arboricola , ATCC 20868, is thawed and used to inoculate 54 milliliters of KF seed medium which is then incubated for 3 days at 25° C at 220 rpm on a rotary shaker with a throw of 5 cm to produce seed culture.

Two milliliter portions of the seed culture are inoculated into a 250-milliliter Erlenmeyer flask containing 50 milliliters of TG103 medium and incubated for 5 days at 25° C with shaking.

At the end of this period, the fermentation broth is harvested. At harvest, the liquid culture is centrifuged to separate the cells from the culture supernatant. The cells are mixed with a 20 ml portion of methanol and the extraction is allowed to proceed overnight at room temperature. The solids are removed by centrifugation, and the methanolic extract is then injected onto a Beckman 5 µ "Ultraphere" (Beckman Engineering) column (6 mm x 25 cm) which is eluted isocratically with acetonitrile/water (48/52) at a flow rate of 0.75 ml/min. The effluent is monitored at 210 nm, and 0.25 ml fractions are collected in 96-well microtiter dishes.

The solvent is removed from the dishes *in vacuo* , and the dishes are assayed for bioactivity by placing in each well a yeast extract, peptone, dextrose (YEPD) broth of the composition below which has been seeded with *Candida albicans* MY1028 and incubating overnight at 37° C. Compound IA, IB, IC and Compound X are produced.

| | grams/liter |
|---|---|
| Bacto yeast extract | 10 |
| Bacto peptone | 20 |
| Dextrose | 20 |
| no pH adjustment | |

## Example IV

Fifty milliliters of KF seed medium in a 250 milliliter unbaffled Erlenmeyer flask is inoculated with a frozen vial of *Z. arboricola* , and shaken at 220 rpm at 25° C for 72 hours. Two milliliters of this seed is used to inoculate 50 ml of KF medium and incubated for 72 hours. The second stage is used to inoculate the production medium.

Forty milliliters of S-6 medium in 250 milliliter unbaffled Erlenmeyer flask are inoculated with 2 milliliters of seed and the flasks shaken at 220 rpm at 25° C for 14 days. A total of 125 flasks are pooled to obtain 5 liters of final broth.

The broth is extracted with an equal volume of methanol and the extract centrifuged. The supernatant is diluted to a final volume of 50 percent aqueous methanol and adsorbed onto a SP-207 column. The column is then washed with three column volumes of 50 percent aqueous methanol and the activity eluted with six column volumes of 75 percent aqueous methanol. An analytical HPLC and a bioassay with *C. albicans* MY 1028 are used to identify bioactive fractions which are combined and then separated by chromatography.

The methanol solution is diluted with water to obtain 50:50 methanol/water and adsorbed onto "Zorbax" ODS column (pre-equilibrated with 50:50 methanol/water)at 20 ml/min. The column is then eluted with 45:55 acetonitrile/water at 20 ml/min. twenty milliliter fractions are taken and the chromatography monitored via UV at 210 nm, with *C . albicans* MY 1028 and analytical HPLC. Compound I and Compound X are found to be produced.

## Example V

A culture of a mutant of *Z. arboricola* on slants of potato dextrose agar medium (Difco) maintained/as MF 5416 in the Merck Culture Collection was employed in the fermentation. A plug of this slant was inoculated into 20 milliliters of KF medium in a 250 milliliter unbaffled Erlenmeyer flask and the flask shaken at 220 rpm and at 25° C for five days. Two milliliters was used to inoculate 40 milliliters of S-6 medium in a 250 milliliter unbaffled Erlenmeyer flask and the flasks shaken at 220 rpm at 25° C for 14 days. A total of 40 flasks were similarly prepared. The contents of the flasks were pooled to obtain a volume of above 1.5 liters. A 20 milliliter sample was analyzed by HPLC and found to contain Compound IC.

1500 milliliters of methanol was added and the mixture stirred for several hours and then filtered. The spent cells were extracted twice with 2 liters of methanol and the extracts were combined to obtain 4200 milliliters. It was diluted with an equal volume of water and adsorbed to 400 milliliters of SP-207 "Diaion" column (pre-equilibrated with $MeOH/H_2O$ 1:1) at 40 ml/min. The column was washed with 400 milliliters of $MeOH/H_2O$ 1:1 and eluted with 100 percent methanol. Two hundred milliliter eluates were monitored by analytical HPLC. Fractions 2-12 were combined to obtain 2 liters of product rich material. This was diluted with 2 liters of water and adsorbed to 400 milliliters of "Diaion" HP-20 column (pre-equilibrated with methanol/water (1:1)). The column was then washed with 800 milliliters of 65% methanol and eluted with 100 percent methanol. Five fractions of 200 milliliters each were combined and concentrated to dryness to obtain 500 milligrams of residue.

Ninety-eight milligrams of this residue was intimately admixed with 4 milliliters of 84 percent methanol in water and chromatographed (DuPont "Zorbax" ODS 21.2 mn x 25 cm 8 µm semi-preparation HPLC column). The initial solvent system was methanol/water (68/32) at 20 ml/min and the effluent monitored via UV at 210 nm and analytical HPLC of the fractions. The mobile phase composition was adjusted twice just to methanol/water (70/30) and than to methanol/water (75/25). Fractions 65-67 were combined and concentrated to obtain 12.1 mg of a compound identified as Compound IC.

## Example VI

1000 compressed tablets each containing milligrams of Compound I are prepared from the following formulation:

| | Grams |
|---|---|
| Compound IB | 500 |
| Starch | 750 |
| Dibasic calcium phosphate hydrous | 5000 |
| Calcium stearate | 2.5 |

The finely powdered ingredients are mixed well and granulated with 10 percent starch paste. The granulation is dried and compressed into tablets.

## Example VII

1000 hard gelatin capsules, each containing 210 milligrams of Compound I are prepared from the following formulation:

| | Grams |
|---|---|
| Compound IA - Compound IC (1:1) | 500 |
| Starch | 750 |
| Dibasic calcium phosphate hydrous | 5000 |
| Calcium stearate | 2.5 |

The finely powered ingredients are mixed well and granulated with 10 percent starch paste. The granulation is dried and compressed into tablets.

## Example VIII

1000 hard gelatin capsules, each containing 210 milligrams of Compound IC are prepared from the following formulation:

| Compound | Grams |
|---|---|
| Compound IC | 500 |
| Starch | 250 |
| Lactose | 750 |
| Talc | 250 |
| Calcium stearate | 10 |

A uniform mixture of the ingredients is prepared by blending and used to fill two-piece hard gelatin capsules.

Example IX

1000 hard gelatin capsules, each containing 210 milligrams of Compound IB are prepared from the following formulation:

| Compound | Grams |
|---|---|
| Compound IB | 500 |
| Starch | 250 |
| Lactose | 750 |
| Talc | 250 |
| Calcium stearate | 10 |

A uniform mixture of the ingredients is prepared by blending and used to fill two-piece hard gelatin capsules.

Example X

250 milliliters of an injectable solution are prepared by conventional procedures having the following formulation:

| | |
|---|---|
| Dextrose | 12.5 grams |
| Water | 250 milliliters |
| Compound IB | 400 milligrams |

The ingredients are blended and thereafter sterilized for use.

Example XI

250 milliliters of an injectable solution are prepared by conventional procedures having the following formulation:

| | |
|---|---|
| Dextrose | 12.5 grams |
| Water | 250 milliliters |
| Compound IC | 400 milligrams |

The ingredients are blended and thereafter sterilized for use.

Example XII

An ointment suitable for topical application may be prepared by intimately dispersing 13 milligrams of Compound IA in 1 gram of commercially available polyethylene/hydrocarbon gel.

## Claims

1. A compound having the formula

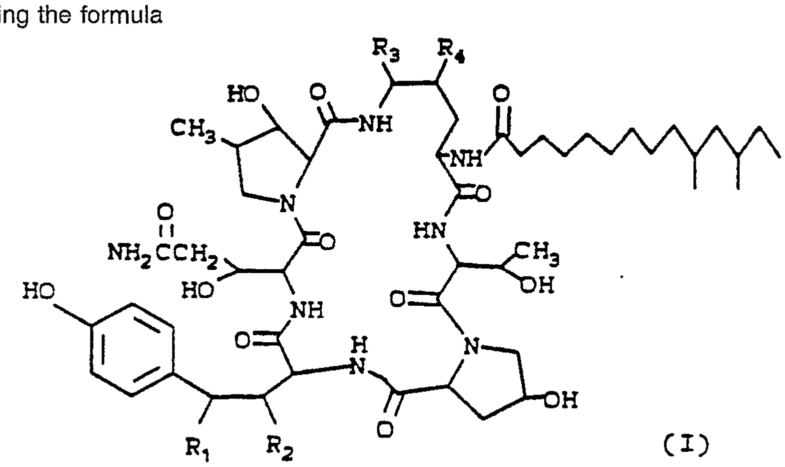

(I)

wherein $R_1$, $R_2$, $R_3$, and $R_4$ are hydrogen or hydroxy and are selected from the following compounds:
(1) $R_1$, $R_3$ and $R_4$ are hydroxy and $R_2$ is hydrogen;
(2) $R_1$, $R_2$, and $R_4$ are hydrogen and $R_3$ is hydroxy; and
(3) $R_1$, $R_2$, $R_3$ and $R_4$ are hydrogen.

2. A compound according to Claim 1 in which $R_1$, $R_3$ and $R_4$ are hydroxy, $R_2$ is hydrogen, and the compound is 1-oxotetradecyl)ornithine]-4-[4-hydroxy-4-(4-hydrophenyl)-2-aminobutanoic acid]-5-(3-hydroxglutamine)echinocandin B.

3. A compound according to Claim 1 in which $R_3$, is hydroxy, $R_2$, $R_3$ and $R_4$ are hydrogen and the compound is named 1-[5-hydroxy-N[2](10,12-dimethyl-1- oxotetradecyl)ornithine]-4-[4-hydroxy-4-(4-hydroxyphenyl)2-aminobutanoic acid]-5-(3-hydroxyglutamine)echinocandin B.

4. A compound according to Claim 1 in which $R_1$, $R_2$, $R_3$ and $R_4$ are hydrogen and the compound is named 1-[N[2]-(10,12-dimethyl-1-oxotetradecyl)ornithine]-4-[4(-4-hydroxyphenyl)2-aminobutanoic acid]-5-(3-hydroxyglutamine)echinocandin B.

5. A composition comprising the compound of Claim 1 in admixture with a pharmaceutically acceptable carrier.

6. A composition according to Claim 5 which is useful for inhibiting fungal growth.

7. A composition according to Claim 5 which is useful for inhibiting or alleviating Pneumocystis carinii infections.

8. A non-medical method for inhibiting fungal growth comprising applying to the area where growth is to be controlled an antifungally effective amount of a compound of Claim 1.

9. A method according to Claim 8 wherein the fungus is Candida albicans .

10. A method according to Claim 8 wherein the pathogen is Candida parapsilosis .

11. The use of the compound of Claim 1, for the preparation of a medicament useful for the treatment of or

for the prevention of Pneumocystis carinii infections in mammals.

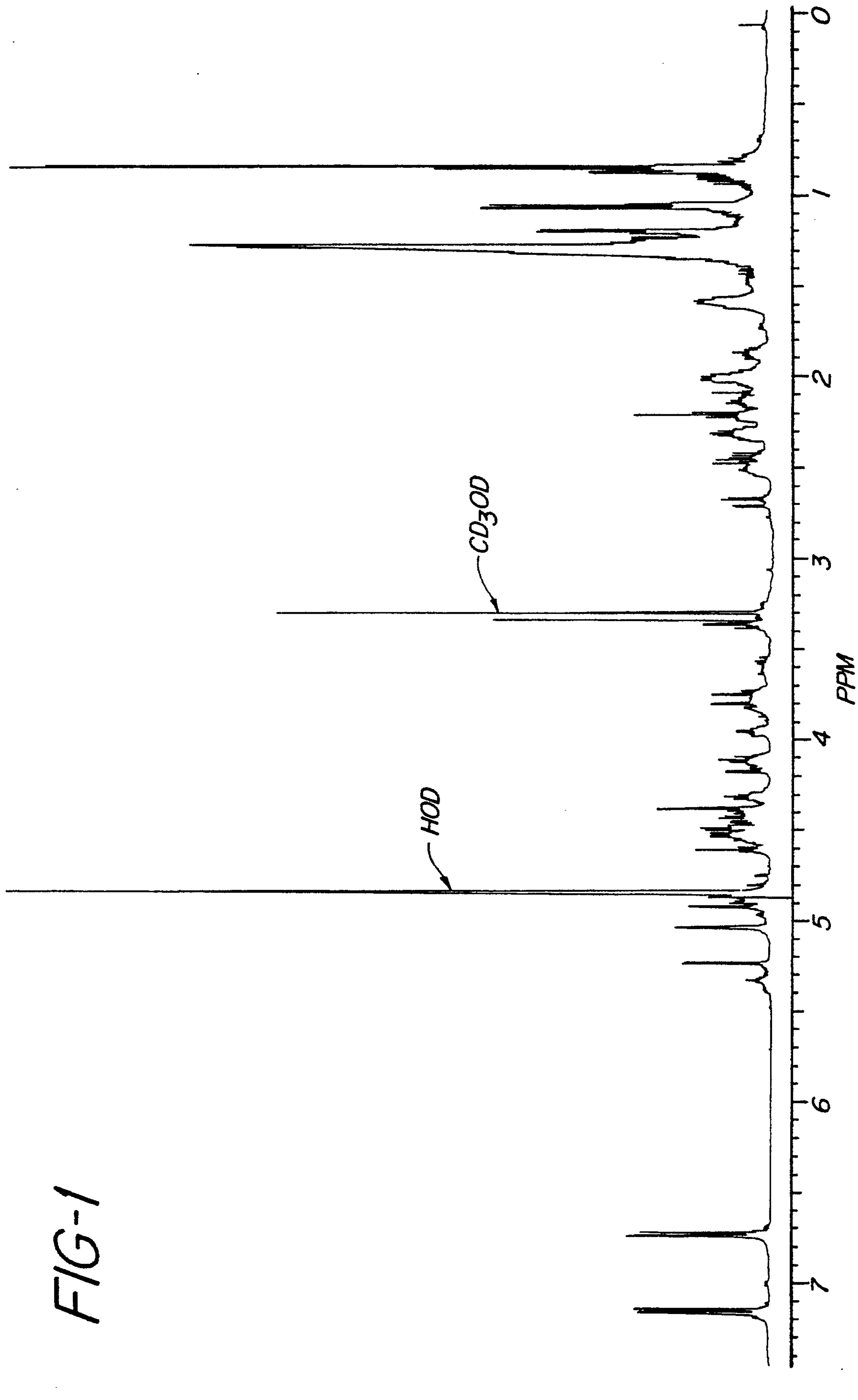


FIG-1

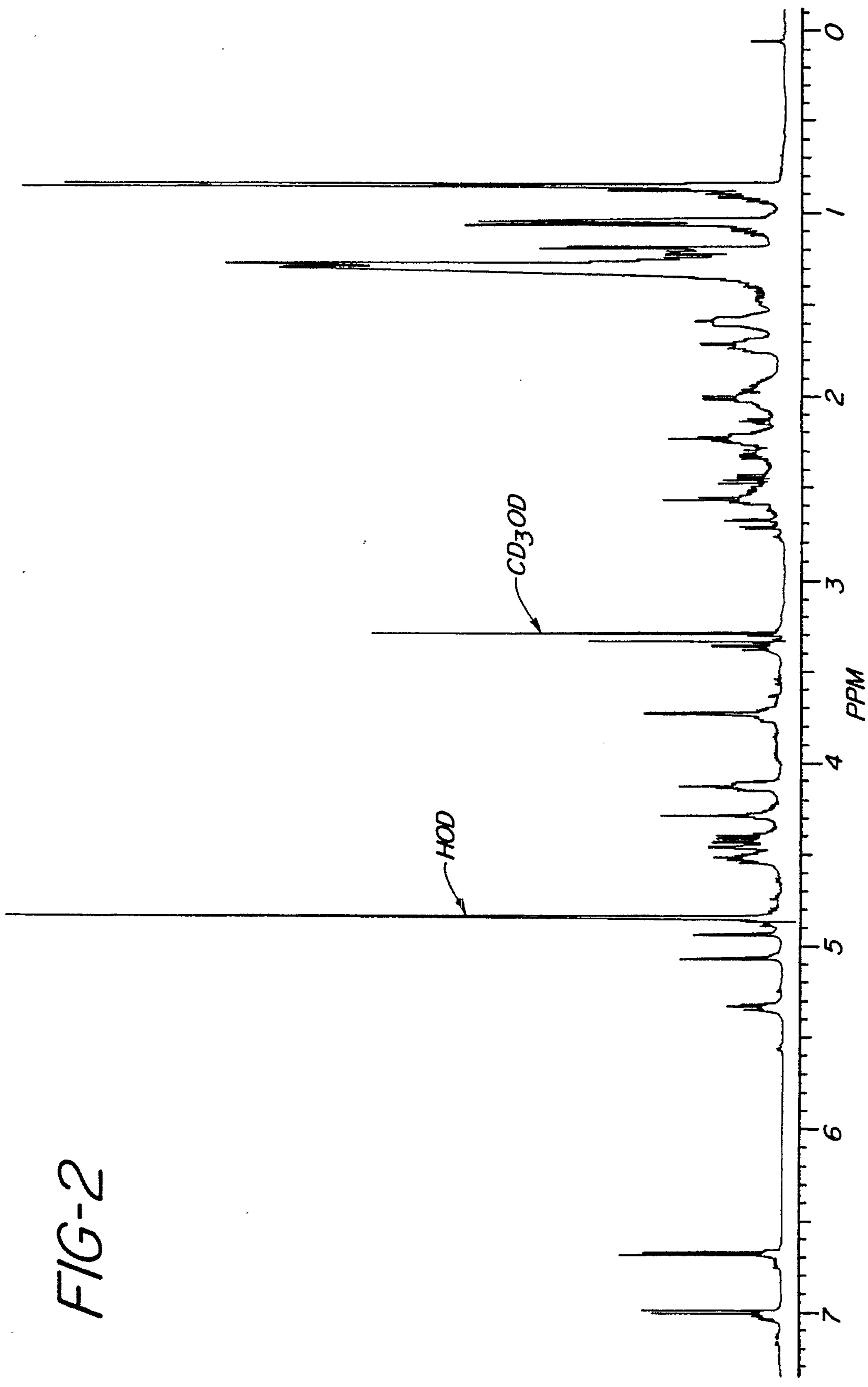


FIG-2

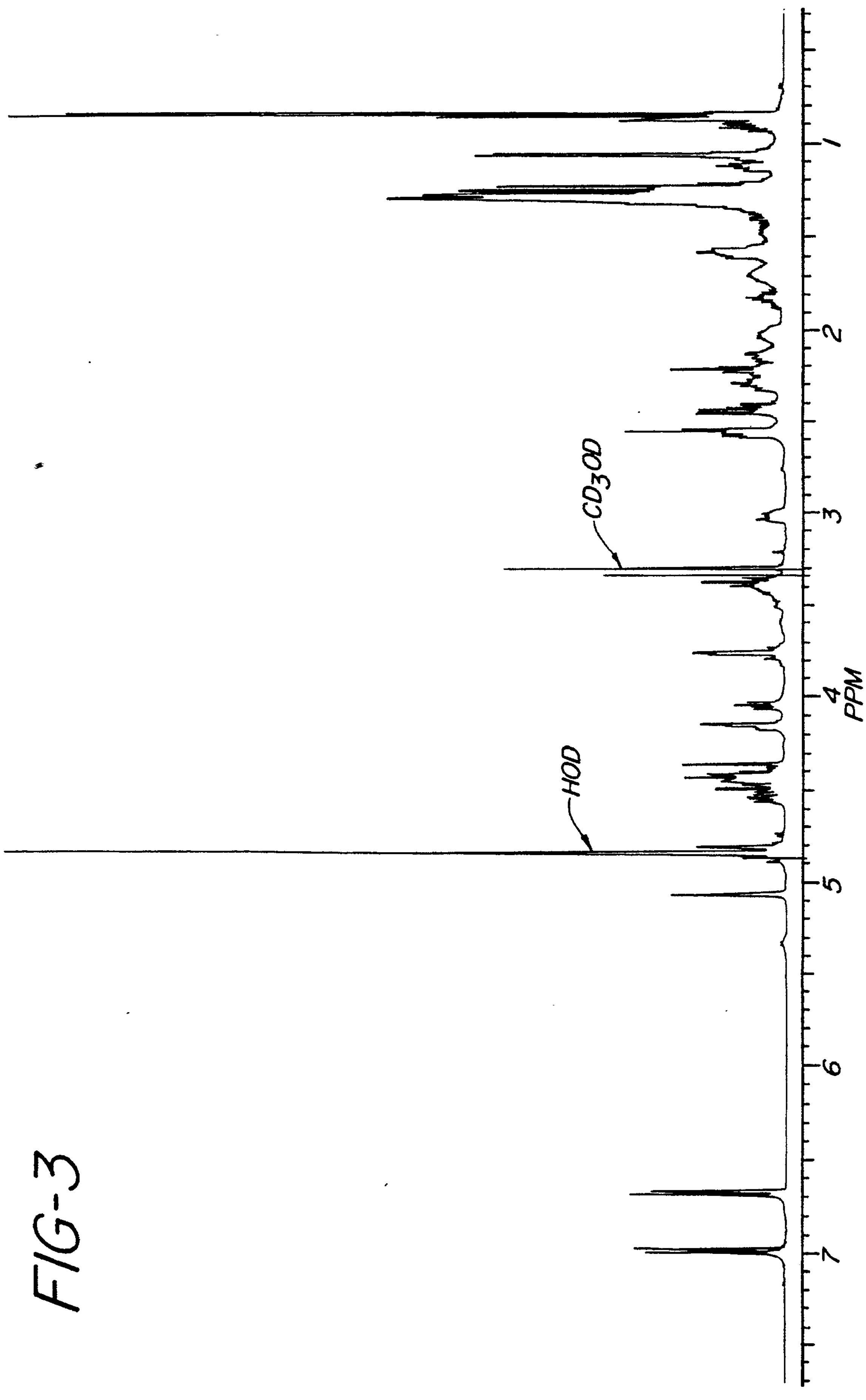


FIG-3

European Patent Office

# EUROPEAN SEARCH REPORT

Application Number

EP 90 30 7125

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP-A-0 359 529 (MERCK & CO., INC) * Pages 1,2; claims * | 1-11 | C 07 K 7/56<br>A 61 K 37/02 |
| A | EP-A-0 311 193 (MERCK & CO., INC.) | | |

TECHNICAL FIELDS SEARCHED (Int. Cl.5)

C 07 K
A 61 K

The present search report has been drawn up for all claims

| Place of search | Date of completion of the search | Examiner |
|---|---|---|
| THE HAGUE | 03-10-1990 | NOVOA Y SANJURJO M.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)